# EUROPEAN PATENT SPECIFICATION

(11) **EP 3 644 941 B1**
(45) Date of publication and mention of the grant of the patent: **18.11.2020**
(21) Application number: 17902578.8
(22) Date of filing: 26.09.2017
(51) Int. Cl.: A61K 8/25, A61K 8/26, A61Q 17/04, A61K 8/19

(54) **A PHOSPHORUS BASED SUNSCREEN CONVERTING ULTRAVIOLET RADIATION TO RED WAVELENGTHS**
SONNENSCHUTZMITTEL AUF PHOSPHORBASIS ZUR UMWANDLUNG VON ULTRAVIOLETTSTRAHLUNG IN ROTE WELLENLÄNGEN
ÉCRAN SOLAIRE À BASE DE PHOSPHORE CONVERTISSANT UN RAYONNEMENT ULTRAVIOLET EN LONGUEURS D'ONDE ROUGES

(30) Priority: 11.08.2017 TR 201711917
(43) Date of publication of application: 06.05.2020
(73) Proprietor: Dokuz Eylul Üniversitesi Rektorlugu, 35210 Izmir (TR)
(72) Inventor: ERTEKIN, Kadriye, 35160 Izmir (TR)
(74) Representative: Kayahan, Senem
(86) International application number: PCT/TR2017/050448
(87) International publication number: WO 2019/032059

(56) References cited:
- WO-A1-2016/063847
- WO-A2-2004/058209
- US-A1- 2003 032 192
- US-A1- 2014 161 850
- US-A1- 2014 161 883

## Description

### FIELD OF THE INVENTION

Present invention is related to a phosphor based highly photo-stable sunscreen composition to protect and improve the skin from the UV radiation of the sun with a different mechanism from the previously commercialized products. More specifically, the phosphor based active ingredients absorb the UV fraction of the electromagnetic radiation, meanwhile, convert the UV radiation to the red wavelengths which is highly beneficial for the skin.

### BACKGROUND OF THE INVENTION

The sun emits radiation over a wide range of wavelengths at varying intensities comprising the ultraviolet (UV), visible (Vis) and infrared (IR) components. The UV fraction of the sunlight constitutes about 9.0 % of the total radiation of the Sun and can further be categorized as UV-A (315 to 400 nm), UV-B (280 to 315 nm), and UV-C (100 to 280 nm). The atmosphere filters almost all the most energetic fraction; UV-C but not the UV-A and the UV-B. On the other hand, human beings living on earth are inevitably exposed to sunlight. Therefore, exposure to sunlight will have a widespread effect.

The UV-A penetrates the upper layers of the skin and can cause undesired photo-toxic effects activating melanin pigment. The UV-B, with a shorter wavelength, is mainly effective on epidermis. The overall UV radiation can cause a lot of undesired short and long-term effects including formation of reactive oxygen species (or more generally free radicals) triggering the production of some secondary chemicals like collagenase, DNA damage, moderate skin burns, edema, photo-aging, hyperpigmentation and skin cancer. It is therefore essential to effectively protect the skin from the sun to minimize the undesired effects. Today a wide variety of sun-protective products have been commercialized and patented. However, further research on sunscreens is needed to develop more efficient and photo-stable formulations considering the side effects of the existing chemical content of the available products.

### Active sunscreen ingredients

Sunscreens are believed to be indispensable tools in providing photo-protection against the harmful effects of the UV radiation. Today a wide variety of formulations offered for protection of skin against the solar UV-A and UV-B. Most of them contain antioxidants, vitamins, thickeners, emulsifiers, preservatives, polymers and cross linkers and many other additives along with UV-filters to improve the cosmetic properties of the product. However, the basic role of a sunscreen is to provide the best protection from the UV. The UV filters can be classified in two main groups; "absorbing" organic molecules and, "absorbing or reflecting" pigment type inorganic materials.

In this context, a number of reports of the prior art describe the use of both types of UV filters to promote the protection efficiency of the sunscreen compositions. For example, the "organic molecules" utilized for this purpose mainly contain an aromatic ring structure or fused rings conjugated with a carbonyl group.

Some of them are para-aminobenzoates (PABAs) including p-aminobenzoic acid, oxyethylene, p-aminobenzoate, 2-ethylhexyl p-dimethylaminobenzoate, ethyl N-oxypropylene p-aminobenzoate, and glycerol p-aminobenzoate, benzophenones including 2,4-dihydroxybenzophenone, 2,2,4,4'-tetrahydroxybenzophenone, 2,2'-dihydroxy-4,4'dimethoxybenzophenone, 2-hydroxy-4-n-octoxybenzophenone, 2-hydroxy-4-methoxy-4'-methoxybenzophenone, salicylates covering 4-isopropylbenzyl salicylate, anthranilate and camphor derivatives and dibenzoyl methanes. Except that of them, 3-(4'-trimethylammunium)-benzyliden-bornan-2-one methylsulfate, cinnemates including 2-ethylhexyl 4-methoxycinnamate, methyl diisopropylcinnamate, isoamyl 4-methoxycinnamate, diethanolamine 4-methoxycinnamate and 2-hydroxy-4-methoxybenzophenone, 2-hydroxy-4-methoxybenzophenone-5-sulfonate, -(2-oxoborn-3-ylidene)-tolyl-4-sulfonic acid and soluble salts, 3-(4'-sulfo)benzyliden-bornan-2-one and soluble salts, 3-(4'methylbenzylidene)-d,l-camphor and 3-benzylidene-d,l-camphor, can be encountered. Some examples of dibenzoyl methane derivatives other than avobenzone include (2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4,4'-dimethoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane and 2,6-dimethyl-4-tert-butyl-4'-methoxydibenzoylmethane, or their mixtures [Cosmetic compositions comprising a structuring agent, silicone powder and swelling agent, US 7749524 B2]

While the benzophenone derivatives, dibenzoyl methanes and anthranilates principally absorbing the UV-A fraction of the radiation, the cinnamates, camphors, PABA derivatives and salicylates absorb the UV-B [ARC Handbooks of Cancer Prevention, Volume 5: Sunscreens]. Sometimes producers use bi-functional organic molecules like phenylbenzimidazole sulfonic acid, octocrylerie, ethylliexyl triazone, 5-Methyl-2-pheriylbenzoxazole and others, or, miscellaneous forms of the above-mentioned molecules in a formulation in order to provide full protection against the whole UV radiation.

On the other hand, number of the pigment type filters is more limited and titanium dioxide and zinc oxide are the most convenient examples of them. Sub- micron size particles and ultrafine powders of the inorganic chemical absorbers of Ti02 and ZnO exhibit superior sun-protection ability with respect to the micro-scale forms. Melanin should also be mentioned among the pigment forms as a biological sunscreen agent.

Today both types of filters (organics and inorganics) have effectively been used as active ingredients in sun protective formulations. However, the long-term side effects of them is a research interest and a number of studies have been published today. Additionally, a number of controversies have developed regarding their safety and efficacy. Therefore, production of an effective and non-toxic sunscreen formulation is still a challenge and efforts in this direction is still ongoing.

Most of the UV-filters are compounds capable of penetrating the skin barrier and, furthermore, they are bio-accumulative. Due to the widespread application of these compounds in many daily-use products accumulation in environment is becoming another issue of great concern. The experimental studies reveal skin absorption of organic filters. When adults applied a sunscreen formulation consisting of benzophenone-3, 4-methylbenzylidene camphor and octyl methoxycinnamate, 3-4 h after application maximum plasma concentrations of 200, 20, and 10 ng/mL for females and 300, 20 and 20 ng/mL for males have been measured, respectively [N.R. Janjua, B. Kongshoj, A.M. Andersson, H.C. Wulf, Sunscreens in human plasma and urine after repeated whole-body topical application, J. Eur. Acad. Dermatol.Venereol. 22 (2008) 456-461].

Significant concentrations of highly lipophilic sunscreen component; 4-methylbenzylidene camphor was measured in human tissues, including placenta [I. Jiménez-Díaz, J.M. Molina-Molina, A. Zafra-Gómez, O. Ballesteros, A. Navalón, M. Real, J.M. Sáenz, M.F. Fernández, N. Olea, Simultaneous determination of the UV-filters benzyl salicylate, phenyl salicylate, octyl salicylate, homosalate, 3-(4-methylbenzylidene) camphor and 3-benzylidene camphor in human placental tissue by LC-MS/MS. Assessment of their in vitro endocrine activity, J.Chromatogr. B Analyt. Technol. Biomed. Life Sci. 936 (2013) 80-87.].

Repeated (4 days) topical applications of 4-methylbenzylidene camphor resulted in urine concentrations and plasma levels of 4 and 18 ng/mL, respectively [N.R. Janjua, B. Kongshoj, A.M. Andersson, H.C. Wulf, Sunscreens in human plasma and urine after repeated whole-body topical application, J. Eur. Acad. Dermatol.Venereol. 22 (2008) 456-461]
Interaction of organic sunscreen 4-Methylbenzylidene-camphor with estrogen receptors in human and rat cells has been subject of another investigation [Mueller SO, Kling M, Arifin Firzani P, Mecky A, Duranti E, Shields-Botella J, Delansorne R, Broschard T, Kramer PJ, Activation of estrogen receptor alpha and ERbeta by 4-methylbenzylidene-camphor in human and rat cells: comparison with phyto- and xenoestrogens, Toxicol Lett. 2003, 30;142(1-2):89-101].

Neurotoxic effects of several organic filters of octyl methoxycinnamate, benzophenone-3 and -4,4-methylbenzylidene camphor, 3-benzylidene camphor and octocrylene, and two inorganic filters; zinc oxide and titanium dioxide have been discussed in a recent study [Joanna A. Ruszkiewicza, Adi Pinkasa, Beatriz Ferrera, Tanara V. Peresa, Aristides Tsatsakisb, Michael Aschnera, Neurotoxic effect of active ingredients in sunscreen products, a contemporary review, Toxicology Reports 4 (2017) 245-259].

Different toxicological studies conducted in animals suggest that some organic UV filters have significant estrogenic and anti-thyroid effects. Small TiO2 particles have also been shown to have serious effects on the mitochondrial function, altering 85 biochemical metabolites, many of which are associated with the cellular stress response [P. Tucci, G. Porta, M. Agostini, D. Dinsdale, I. Iavicoli, K. Cain, A. Finazzi-Agro, G. Melino and A. Willis, Cell Death Dis., 2013, 4, e549.]

Both ZnO and TiO2 exhibit UV-induced photocatalytic activities, which induces reactive oxygen species and damages human cells [A Liou JW, Chang HH. Bactericidal effects and mechanisms of visible light responsive titanium dioxide photocatalysts on pathogenic bacteria. ArchImmunol Ther Exp (Warsz). 2012; 60:267-75., Yin B, Zhang S, Zhang D, Jiao Y, Liu Y, Qu F, et al. ZnO Film Photocatalysts. J Nanomaterials 2014; Hashimoto K, Irie H, Fujishima A. TiO2 Photocatalysis: A Historical Overview and Future Prospects. Jpn J Appl Phys. 2005; 44:8269-85].

Not only the skin penetration but also the photo-stability of the UV filters are discussion topics. Evidences show that UV induced sunscreens undergo degradation processes forming free radicals, which reduces skin protection and consequently induces skin oxidative stress with the depletion of antioxidant defense mechanisms such as reduced glutathione and superoxide dismutase. In this regard, the biological effects of the commercially available sunscreen formulations upon irradiation should be considered.

Several patents have been assigned, including dibenzoylmethane derivatives [US6444195 B1: Sunscreen compositions containing a dibenzoylmethane derivative, WO2000057850 A1: Photostable sunscreen compositions containing dibenzoylmethane derivative, e.g., parsol® 1789, and diesters or polyesters of naphthalene dicarboxylic acid photostabilizers and enhancers of the sun protection factor (spf) ], benzophenones [US 3670074 A : Sunscreen formulation containing triethanolamine neutralized 2-hydroxy-4-methoxy-benzophenone-5-sulfonic acid, US 7001592 B1: Sunscreen compositions and methods of use, US 7416719 B2 : Sunscreen compositions, CA 2468228 A1: Stabilization of sunscreens in cosmetic compositions ] zinc oxide, [US 6464965 B1: Sunscreen composition, US 20060280702 A1: Stable sunscreen compositions containing zinc oxide] titanium dioxide [WO 1996037560 A1: Titanium dioxide hydrogel and sunscreen composition containing it CA 2741872 C : Sunscreen compositions for application to plants], or combined forms of zinc oxide and titanium dioxide [US 20110070174 A1: Benzoyl Peroxide and Sunscreen Agent Combination] , ferulic acid amides and salts of polyvalent metals. In the light of the above discussion, there is a necessity for production of less toxic and more stable sunscreen formulations. Patent document WO 2016/063847 discloses a skin care UV screening composition.

On the other hand, excellent absorption and tunable emission characteristics of the photoluminescent inorganic phosphors are well known. Different phosphors have different emission abilities and when excited with the ultraviolet light or visible, a high-quality light spectrum is produced. Especially the materials doped with rare-earth ions have attracted great attention because of their high UV absorption capacity, and emission abilities at further wavelengths. The use of inorganic phosphors has found wide application in sensor technologies, scintillators, lighting, LED and display technologies, lasers and similar products, but its use in medical studies and pharmacy has been limited to cell imaging efforts. U. S.

Patent no 8852616 B2 issued to Bickford et. al. on December 2012 disclose the use of some Near-infra-red (NIR) light emitting phosphors due to the skin stimulating healing and/or regenerative properties. The phosphors include MgSiO3:Eu2+, Dy3+, Mn2+; Ca0.2Zn0.9Mg0.9Si2O6, doped with Eu+2, Dy+3, Mn2+; SrAl2O4:Eu2+, Dy3+, Er3+; La3Ga5Ge3O14:Cr3+, with or without co-dopants such as Li+, Zn2+, Ca2+, Mg2+ and Dy3+; Ln3Ga2Ge4O14:Cr3+(Ln=Y, Gd, La or Lu); LiGa5O8:Cr3+; M3Ga2Ge4O14:Cr3+ (M=Sr or Ca); La3Ga5SiO14:Cr3+; La3Ga5.5Nb0.5O14:Cr3+; La3Ga5GeO14:Cr3+; Gd3Ga5O12:Cr3+; and ZnxGayGezO(x+(3y/2)+2z):tCr3+, with or without co-dopants. However, the offered formulation has not been considered as a sunscreen. To the best of our knowledge, no work has been reported using phosphors as active sunscreen agents.

### Other ingredients in sunscreen compositions

Emollients are cosmetic ingredients that soften the skin and seal in moisture, creating an occlusive, protective barrier on the surface. They may be classified as emollient esters and oils, wax materials, hydrocarbons, higher fatty acids and alcohols, and silicones.

The naturally occurring esters, more specifically triglycerides and lanolin have extensively been used as additives in cosmetics including the sunscreens. Olive oil, castor oil, sesame oil, sunflower, wheat germ, pumpkin seed, almond oil, jojoba and coconut oil are examples of the first group. The major fatty acids in olive oil are: Oleic Acid (C18:1), a mono unsaturated omega-9 fatty acid. It makes up 55 to 83% of the olive oil. Linoleic Acid (C18:2), a poly unsaturated omega-6 fatty acid that makes up about 3.5 to 21% of the olive oil. Palmitic Acid (C16:0), a saturated fatty acid that makes up 7.5 to 20% of the olive oil. Stearic Acid (C18:0), a saturated fatty acid that makes up 0.5 to 5% of the olive oil. Linolenic Acid (C18:3) (more specifically alpha-Linolenic Acid), a poly unsaturated omega-3 fatty acid that makes up 0 to 1.5% of the olive oil. Similarly, the sesame oil contains large amount of oleic (35-50%) and linoleic acid (35-50%), and less or trace amounts of palmitic stearic, linoleic and eicosenoic acids. Additionally, penetrates the skin easily. Bee wax (Ceryl 16-hydroxypalmitate, triacontanyl palmitate, myricyl palmitate, cetyl palmitate) and candellilla wax (Hydrocarbone(C31H64) and C16-C34 fatty acids) are examples of the waxy materials. Parafine and squalane and cetyl-stearyl alcohol are examples of hydrocarbons and alcohols, respectively. Lauric, myrisitc, palmitic, stearic and iso-stearic acids can be encountered as the examples of the higher fatty acids. Glyceryl Stearate, Lecithin, Polyglyceryl Oleate, Glycol Distearate, Shea Butter Glycerides, PEG-7 Glyceryl Cocoate, ethoxlyated polyethylene glycol ester made from castor oil, and C18 stearyl alcohol and C16 cetyl alcohol may be used as emulsifiers in sunscreen compositions.

### Antioxidants

Antioxidants: It is accepted that free radical formation has an important effect on skin aging. Free radicals also known as "reactive oxygen species" are hosts for unpaired electrons and are harmful for a variety of cellular structures like lipids, proteins and the DNA. The UV light from the sun estimated to generate DNA damage through a similar mechanism forming photochemical reactions. Antioxidants may prevent or delay some types of cell damage. Therefore, the "antioxidants" are widely used in cosmetic products. The antioxidants mentioned here are the ones that fight against the free radicals by their topical effects. Examples of antioxidants include natural origin plant extracts, especially flavonoids, butylated hydroxy toluene, caffeine, caffeic acid, vitamin C (ascorbic acid) and its stabilized forms 'alanine' some algae, vitamin E (alpha-tocopherol), certain amino acids (alanine, arginine, asparagine ..), anthocyanidins, some components of argan oil, asparagine and derivatives, beta-glucans, polysaccharides, beta carotene, chitosan, cinnamon, coenzyme Q10, curcumin, gamma linolenic acid (GLA), L-cysteine, glutathione, hesperidin, green tea extract, hibiscus (rosehip) extract, linoleic acid, lycopene, lutein, palm kernel oil, olive oil, propolis, pumpkin seed oil, spirulina, tannic acid, tea tree oil, and tetrahexyl decyl ascorbate.

### Emulsifiers

In cosmetic preparations, there may be two immiscible liquid phases (oil and water) and each phase usually contains a variety of valuable components. Emulsifiers blend and hold together these ingredients. Some commonly used emulsifiers in skin care are glyceryl stearate, PEG-100 stearate, stearyl alcohol, cetyl alcohol, laureth-23, steareth alcohol, cetyl/PEG/PPG 10 dimethicone, and stearic acid. Fatty acids are key components of emulsifiers due to their miscibility in a variety of natural and synthetic oils.

They may be derived from natural oils such as olive oil, sunflower, coconut, palm, kernel, wheat germ, etc. They are also mentioned as thickening agents to make thin formulations more viscous, sometimes as secondary emulsifiers to prepare stable mixtures of oil and water, and as opacifying agents to make the resulting products look more luxurious.

### Structuring agents

Structuring agents may simply be classified as "aqueous phase structuring agents" and "oil phase structuring agents". They also may be classified in many different ways including the classification according to the occurrence of the material; as: "natural" and "synthetic" .

Hydroxyethyl cellulose, agarose, hyaluronic acid, algin, alginic acid, acacia gum, amylopectin, modified forms of chitin, dextran, cassia gum, cellulose gum, gelatin, pectin, and xanthan gum are examples of naturally occurring forms. Carboxy methyl cellulose, acrylic acid, methacrylic acid, and mixtures, acrylic copolymers, acrylate copolymers and their cross linked forms, various polyethylene glycols (PEGs), and polyglycerins can be encountered as the examples of synthetic polymeric thickeners. Both, aqueous phase and oil phase structuring agents work on the same principle to increase viscosity of the blend to the desired value. The structuring agent should also be an appropriate matrix material for the sunscreen in terms of optical properties and should be compatible with the other ingredients of the formulation.

### BRIEF SUMMARY OF THE INVENTION AND OBJECTIVES

The present invention relates to a phosphor-based, highly photo-protective sunscreen having the potential to overcome the disadvantages of the existing products and bring new advantages to the related art.

The aim of the present invention is to provide a high level protection from the sun light and simultaneously provide a positive effect on the skin with a formulation which is different from the sunscreens known to this day in terms of the "active sunscreen ingredient".

Another object of the invention is to absorb the UV-B and UV-A fractions which is harmful to the skin and to realize a strong and simultaneous conversion of the UV radiation to the beneficial red wavelengths.

Another object of the invention is to absorb the less energetic visible region of the electromagnetic spectrum between 390 and 490 and to convert the red light, by this way create similar benefits on the skin.

A preferred embodiment of the invention is the use of supplements which may be advantageous for the skin as well as the phosphor based active ingredients.

Another preferred embodiment of the invention is the encapsulation of phosphorus-based photo-stable agents in an optically compatible support matrix.

A preferred embodiment of the invention is that the developed composite is compatible with the skin and is persistent and cosmetically suitable.

### FIGURES DESCRIBING THE INVENTION

The figures prepared to better explain the sunscreen formulation developed with this invention are described below.
Figure 1. Time dependent variations in the luminescence intensity of Sr4Al14O25:Eu2+/Dy3+ type afterglow phosphor after irradiation at 400 nm, at room temperature. (Was taken from: S. Demirci, S. Gültekin, S. A. Akalin, Ö. Öter, K. Ertekin, E. Çelik, Materials Science in Semiconductor Processing 31 (2015) 611-6172).
Figure 2 - Schematic representation of penetration depth of the visible and red light in the layers of the skin.
Figure 3. Decay characteristics of the sunscreen formulation (Data were acquired on Mylar substrate after excitation with a microsecond flash lamb at 350 nm, average decay time 31.44 microseconds).
Figure 4. Emission performance of the sunscreen formulation upon excitation at the wavelengths between 380 and 490 nm (Data were acquired on an acetyl cellulose substrate).

### DETAILED DESCRIPTION OF THE INVENTI ON

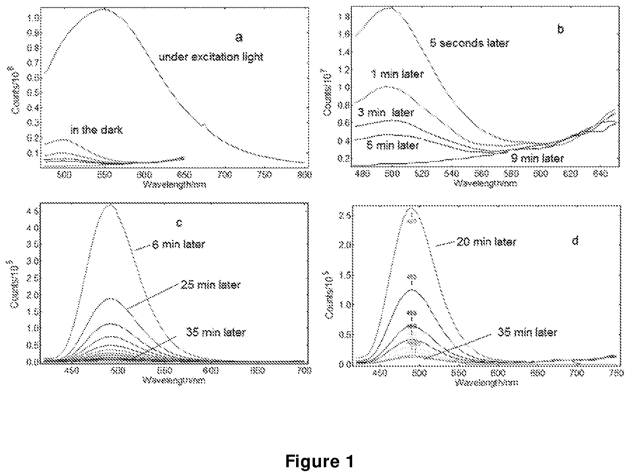

Luminescence is a collective term defining different phenomena where a substance emits light after excitation. If the excitation is performed by the light, the following room temperature emission process is "photoluminescence". The two well-known forms of photoluminescence are fluorescence and phosphorescence. In both cases, the light is absorbed and emission occurs at further wavelengths than the absorbed light. Absorption process occurs over very short time intervals as fast as 10-15 s. Therefore, the material becomes excited. De-excitation to electronic ground state with emission of longer wavelength light, in other terms fluorescence, occurs in 10-9 seconds. This time interval also called as "nanosecond". A phosphorescent material absorbs energy and traps the excited electrons in higher energy states for longer times with respect to the fluorescent species. The durations of the phosphorescence start from milliseconds and extend to time intervals expressed in minutes or even hours. Both; organic dyes and inorganic materials may exhibit effective phosphorescence. Lanthanide complexes of organic compounds or phosphors may exhibit strong emissions in the microsecond and millisecond range. The phosphors emitting in longer time intervals like minutes, hours or days are called "afterglow phosphors". This type of long-term emission of phosphorus seems to be an advantage, but it has been proven by measurements that the intensity of emissions falls exponentially over time (See Fig.1).

Today, phosphors and phosphor blends have been found wide application extending from electronics, LEDs, television and phone screens to medical imaging tools. Therefore, research is focused on development of new phosphors due to the increase in their application spectrum. Many of them have rapidly been patented and/or commercialized. However, there is no data indicating utilization of the phosphors in sunscreen cosmetics. The U. S. Patent (no 8852616 B2) issued to Bickford et. al. on December 2012 is regarding to the utilization of long- persistent luminescence materials. More specifically the subjective invention is directed to cosmetic products for the body, and in particular, for the skin, scalp and hair, which incorporate a material which emits persisting near-infra-red (NIR) light in order to provide long term benefits covering stimulating healing or regenerative properties in the skin. However, this invention does not address sun protection.

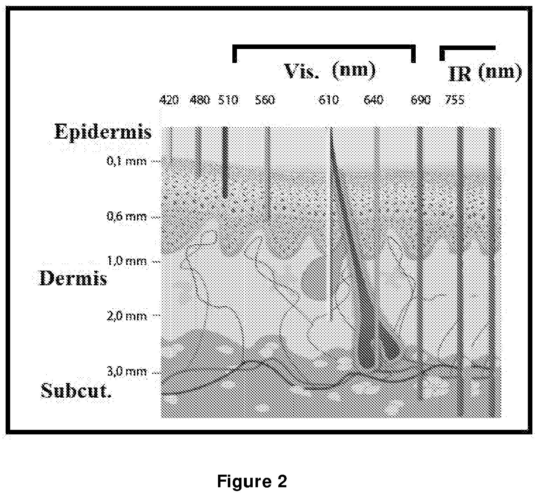

On the other hand, wavelength range of 550-850 nm of the electromagnetic spectrum is known as the red (RED) region. According to the spectroscopists, the near-infrared (NIR) window is in the wavelength range of 700-1350 nm. The spectral range between 600 nm and 1300 nm is known as "tissue optical window" or "therapeutic window" where light has its maximum depth of penetration in tissue. (See Fig. 2). Melanin, hemoglobin, oxyhemoglobin, hemosiderin, bilirubin and carotenoids can absorb the wavelengths centered around the "red light". Mid IR light can be absorbed by water and lipids. Therefore, absorption of the radiation in the therapeutic window may provide some benefits for the skin including stimulation of collagen and/or elastin, enhancement of DNA synthesis, improving the skin texture and repair and rejuvenation of the scars on the skin Journal of Investigative Dermatology, Volume 137, Issue 2, February 2017, Pages 466-474), (Journal of Photochemistry and Photobiology B: Biology Volume 170, May 2017, Pages 197-207). For this reason, commercialized devices equipped with LED or laser sources emitting in the NIR region have been developed to stimulate wound healing, to improve the texture of skin, to reduce the size of pores and depth of wrinkles, and to reduce the cellulite formation.

In the light of the above discussion, this invention reveals structure of a formulation that can absorb and then simultaneously turn the harmful UV radiation into red wavelengths that are entirely useful to the skin and capable of penetrating the skin.

Skin benefits obtainable with the phosphor based constituents arises from the superior absorption and perfect emission abilities of the phosphors which covers the whole UV and extends to 550 nm in terms of the absorption and 550 nm and further wavelengths, in terms of the emission. According to the results of a recent study, beneficial effects of the red light starts by the absorption of the cell constituents like Cytochrome c oxidase and extends to mitochondrial metabolism, including Ca2+and NO levels and the synthesis of the ATP [Photochem Photobiol. 2015 Mar; 91(2): 411-416.]

Potential benefits obtainable with the phosphor based constituents of the present invention also include reduction in formation of reactive oxygen species and consequently the DNA damage, reduction in inflammation; stimulation of synthesis and repair of the collagen or elastin in the skin and a general improvement in the texture of skin.

When applied on the skin, the effect of the product is long lasting unless it is removed.

In contrast to the afterglow phosphors, the utilized phosphors in this formulation do not lose their emission intensities by time.

Any not toxic and photo-stable material which absorbs the solar UV and emits between the 500-1400 nm, may have been used in the formulation of the present invention. The light conversion has been accomplished simultaneously.

The sunscreen product presented by this invention may include, but not limited to phosphors of, GdYEu(MoO4)3 doped by Eu and/or Sm, Mg14Ge5O24 doped by Mn, CaAlSiN3 doped by Eu, or, (Sr,Ca)AlSiN3 doped by Eu, or (LaY)3Si6N11doped by Ce or Lu3Al5O12 doped by Ce, or (LaY)3Si6N11doped by Ce, or their blends. Examples of the phosphors including Eu doped (Sr,Ca)AlSiN3 sold by Nichia and Mitsubishi Chemical Corporation (CASN or SCASN phosphor, or 1113 phosphor) Or Dowa Electronics and Intematix under the brand name of XR6436 red nitride phosphor. The dopants present in the structure of the host defined in this invention may be in the oxidation sates of Eu2+, Sm 2+, Eu3+, Sm 3+, Ce3+ or Mn4+, respectively. Emission occurs after the excitation of the dopants of Eu, Sm Ce or Mn at appropriate oxidation state by the UV or visible light. Host structures of the utilized phosphors may also be responsible from the absorption and following emission processes.

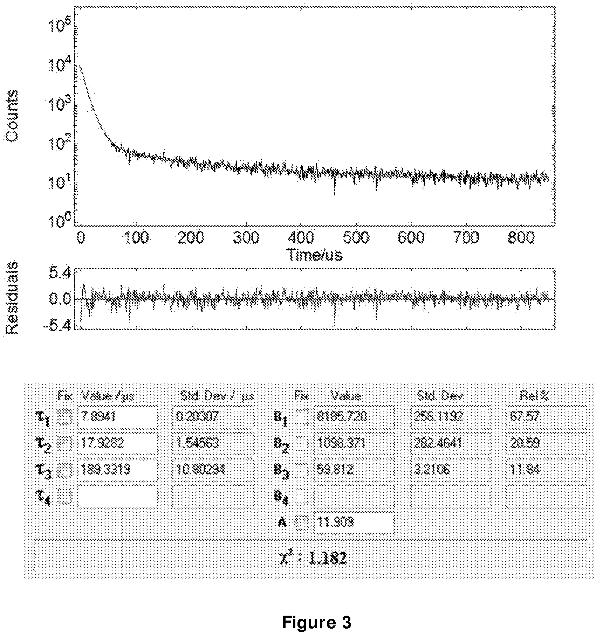

The utilized phosphor based composition can convert the UV radiation to the RED wavelengths, within very short time intervals. Figure 3 reveals decay characteristics of the phosphors used in this formulation. Decay time related data were acquired on Mylar substrate after excitation with a microsecond flash lamb at 350 nm. According to the Figure 3, the phosphors exhibit tri-exponential decay behavior. Average decay time can be stated as 31.44 microseconds. From the Fig.3 it can be concluded that, the utilized phosphors are able to immediately convert the UV radiation to the red light.

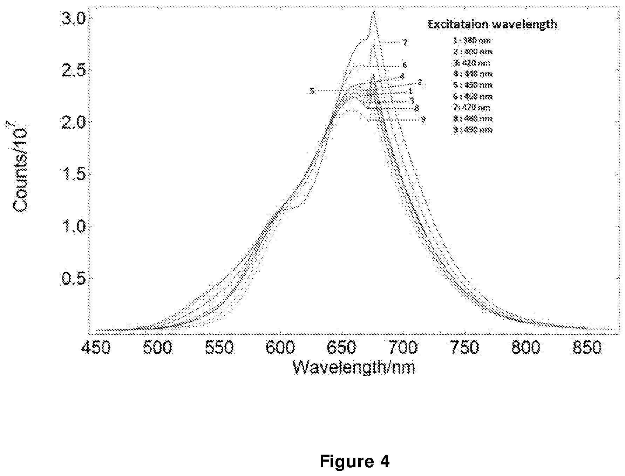

Figure 4 reveals emission performance of the sunscreen formulation upon excitation at different wavelengths between 380 and 490 nm. From the Fig.4 it can be concluded that the emission wavelength is independent from the excitation light for at least between 380-490 nm.

A further aspect of the invention relates to the dimensions of the phosphors, wherein the sizes of the phosphorus may be in the range of 100 nm and - 50.0 µm.

Another aspect of the invention relates to encapsulation of the active ingredients absorbing and emitting light that is dermatologically acceptable and does not limit the performance of phosphors optically. Matrix material may enhance the emission characteristics of the phosphors and may contain other excipient chemicals like emollients, thickeners, antioxidants, fatty acids, etc.

Emollients used as supporting materials in sunscreens other than active ingredients soften the skin while preventing water loss. Examples of these include vegetable oils, mineral oils, sia and cocoa butter, olive oil, coconut oil and fatty acids, lanolin from animal fats, bee wax, emu oil and so on. More technically, these materials are called triglycerides, benzoates, myristates, palmitates and stearates. Suitable emollients used in this invention include, but are not limited to, lipophilic materials such as saturated or unsaturated C6-40 straight or branched chain fatty acids, or saturated or unsaturated C6-40 straight or branched chain fatty alcohols. Examples include oleic acid, linoleic acid, stearic acid, oleyl alcohol, linoleyl alcohol, and the like. In the present invention emollients may be in the range of 0.001 to 30% w/w, preferably from about 0.005 to 25%, more preferably from about 0.01 to 20%. Present invention may include a mono-unsaturated omega-9 fatty acid "oleic acid" in the concentration range of 55 to 83%. Present invention may also include a polyunsaturated omega-6 fatty acid; linoleic acid in the concentration range of 3.5 to 21%. Present invention may include palmitic, stearic and linolenic acids in the concentration range of 7.5 to 20%, 0.5 to 5% and 0 to 1.5%, respectively. In the present invention, water and/or glycerin extracts of the Pistacia lentiscus may be found in the range of 0.01 to 10%. The essential oils were extracted according to the protocols in the Japanese Pharmacopoeia. Distribution of the essential olis of the Pistacia lentiscus in the sunscreen formulation may be as follows: α-pinene (82 %) β-pinene(3.0 %) β-myrcene (2.0%) and linalool(1.5%). Trace amounts of the α-terpineol, p-cymene-8-ol, myrtenal, p-cymene, limonene, (E)-carveol, 2-undecanone, α-and β-caryophyllene, caryphyllene oxide and (E)-Me isoeugenol may be found in the sunscreen composition. The E)-methyl isoeugenol and α-terpineol also exhibits antibacterial efficiency against H. pylori and E. coli, S. Aureus and B. Subtillis [Nat Prod Bioprospect,v.4(4); 2014 Aug 227-231 Chemical Composition of the Essential Oil of Mastic Gum and their Antibacterial Activity Against Drug-Resistant Helicobacter pylori, Tomofumi Miyamoto, Tadayoshi Okimoto, and Michihiko Kuwano].

One type of structuring agent that may be used in the composition comprises orientalis sweet gum which is mainly composed of styrene and less amounts of cinnamyl alcohol and α-pinene as the major components.

Another type of structuring agent that may be used in the composition comprises Stearyl Alcohol. The constituent acts as softener and stabilizer. Stearyl Alcohol is a long chain fatty alcohol, and found in a wide variety of skin care products such as hair conditioners, foundations, moisturizers, skin cleansers and in many others. It acts as a lubricant on the skin surface, which gives the skin a soft, smooth appearance.

The composition may contain one or more emulsifier agents. Cetyl alcohol and glyceryl stearate are the examples of the emulsifiers. If present, the emulsifier may range from about 0.005 to 25%, by weight of the total composition. This emulsifying blend is appropriate for making oil-in-water emulsions.

It may be desirable to include one or more structuring ingredients in the sunscreen compositions of the invention. Structuring agents are ingredients that contribute to formation of an appropriate matrix for the active ingredients. They may be present in an amount sufficient to provide a liquid composition with desired viscosity. Offered ranges of structuring agents may be in the range of 0.1 to 70% by weight of the total composition. These ingredients are most often found in the polymeric form and may be hydrophilic or hydrophobic. They can serve as matrix for active cosmetics components, such as, active sunscreen forms, antioxidants or antimicrobials and other additives preserving their functionality and stability. Examples of the structuring agents are polysaccharides, acrylate type polymers or copolymers, or their cross linked forms, High Molecular Weight Polyglycerins, some Volatile Oils and silicones, non-volatile silicones and oils, silicon waxes, mono-, di-, and tri-esters, vegetable and animal source glyceryl esters and fatty acids. Sodium Carboxymethyl Cellulose (CMC) is a water soluble anionic polymer and functions as a thickening agent, moisture retention agent, texture/body building agent, suspension agent, and binding agent in personal skin-care products and some drugs.

Mid molecular weight Sodium Carboxymethyl Cellulose may be used in the present invention for entrapping and stabilizing the light-emitting phosphor based material.

The present invention may contain additional cosmetically and/or dermatologically beneficial ingredients, including such as described herein below.

Serum or gel forms of skin care products will generally comprise from about 1-99% water, and optionally from about 0.001-30% of an aqueous phase thickening agent. Typical skin creams or lotions comprise from about 4-99% water, 1-90% oil, and from about 0.1 to 20% of one or more surfactants. In the present invention, the water content may range from about 1.0 to 85%, by weight of the total composition.

It may also be desirable to include one or more humectants in the sunscreen composition. Ratio of humectants may range from about 0.001 to 25% by weight of the total composition. A humectant or moisturizer is a substance that attracts and retains water molecules on the layers of skin. Glycerin and glycerol are the most common humectants found in skin care products. Others include some alpha hydroxy acids, panthenol, carboxylic acids, sorbitol, sodium salt of hyaluronic acid, sodium and ammonium lactate, propylene glycol, sodium pyrrolidine, urea, gelatin, and honey. Examples of such sugar type humectants include glucose, fructose, honey, hydrogenated honey, inositol, maltose, mannitol, maltitol, sorbitol, sucrose, xylitol and xylose. Examples of other humectants also include polyethylene and polypropylene glycols (PEGS) such as Lysolecithin, PEG-14 Dimethicone, PEG/PPG-14/4 Dimethicone PEG-7 Glyceryl Cocoate, PEG-6 Caprylic/Capric Glycerides, Polyglyceryl-4-caprate, PEG-10 Sunflower Glycerides Poloxamer 105, Poloxamer 182 Dibenzoate, Dimethicone PEG/PPG-20/23 Benzoate, PEG 26 Glycerine, Propylene Glycol, Laminaria Digitata Extract and others. Most of them are commercially available and sold under different brand names. Examples of them are, Lysolecithin, PEG-14 Dimethicone, PEG/PPG-14/4 Dimethicone PEG-7 Glyceryl Cocoate, PEG-6 Caprylic/Capric Glycerides, Polyglyceryl-4-caprate, PEG-10 Sunflower Glycerides Poloxamer 105, Poloxamer 182 Dibenzoate, Dimethicone PEG/PPG-20/23 Benzoate, PEG 26 Glycerine, Honey extract. Propylene Glycol and Laminaria Digitata Extract and others. The humectants used in the present invention are, panthenol and glycerin. Ratio of the humectants used in this invention may range from about 0.01 to 10% by weight of the total composition.

An emulsifying agent keeps immiscible liquids from separating by increasing the kinetic stability of the mixture. Cetearyl Alcohol, Polyglyceryl-6 Distearate, Sodium Stearoyl Lactylate, Sodium Lauroyl Lactylate and lecithin are examples of the cosmetically avaliable emulsifiers. Among them lecithin is a non-ionic emulsifier bearing 4 phospholipids; phosphatidyl choline, ethanolamin, serin, and inositol. It is capable to bind water and fats and therefore is an emulsifier suited to both, water in oil, and, oil in water emulsions depending on temperature and ratio. These desirable properties make the lecithin an ideal choice for a variety of applications. One of the emulsifiers used in the present invention is, lecithin. Ratio of the emulgator used in this invention may range from about 0.001 to 1.00% by weight of the total composition.

### Antioxidants

After exposure to high levels of the ultraviolet radiation, formation of different types of reactive oxygen species including singlet oxygen, superoxide radicals, and peroxide radicals is possible. These forms may cause damage on cellular constituents of lipids, proteins, and even DNA depending on the dose, and they are considered to be the primary reasons to erythema (sunburn), premature aging of the skin and skin cancers. Antioxidants are involved in the prevention of cellular damage and are also thought to have a role in slowing the aging process. Lycopenes, flavonoids, proanthocyanidins, astaxanthin, beta-carotene and vitamin E (alpha-tocopherol), have been shown among the most powerful antioxidants to fight with the reactive oxygen species. Some plant extracts, bioflovanoids, butylated hydroxytoluene, caffeine, caffeic acid, vitamin C (ascorbic acid) and its stabilized forms, some algae, some amino acids (alanine, arginine, asparagine), glucan, chitosan, cinnamon, coenzyme Q10, curcumin, gamma linolenic acid (GLA), L-cysteine, glutathione, hesperidin, green tea extract (aspartame), some components of arganic oil, asparagine and its derivatives, polysaccharide beta-glucans, Hibiscus extract, linoleic acid, lutein, palm kernel oil, olive oil, propolis, pumpkin seed oil, spirulina, tannic acid, tea tree oil, tetrahexyl decyl ascorbate and many others are the examples of the antioxidants.

The antioxidants used in the present invention may be, retionol, tocopheryl acetate and tannins tri-, tetra-, and pentacyclic triterpene acids. Ratio of the antioxidants used in this invention may range from about 0.01 to 1.00% by weight of the total composition.

## Claims

1. Sunscreen composition **characterized in** comprising at least one phosphor selected form Eu-Sm doped GdYEu(MoO4)3, Mn doped Mg14Ge5O24, Eu-doped CaAlSiN3, Eu doped (Sr,Ca)AlSiN3, Ce doped (LaY)3Si6N11, Ce doped Lu3Al5O12 or their blends and at least one excipient.

2. A photostable sunscreen emulsion composition according to claim 1, wherein the active sunscreen agents comprising (Sr,Ca) AlSiN3 which is doped by Eu, and/or GdYEu(MoO4)3 doped by Eu and Sm ,and, Mg14Ge5O24 doped by Mn, CaAlSiN3 doped by Eu, and/or, (LaY)3Si6N11doped by Ce or Lu3Al5O12 doped by Ce or their blends are immediately activated upon exposure to the sunlight.

3. The sunscreen formulation according to claim 1 wherein the cosmetic excipients selected from natural oils, thickeners, emulsifiers, structuring agents and humectants comprises deionized water, sodium Carboxymethyl Cellulose (CMC), glycerin, cetyl alcohol and glyceryl stearate, Oleic Acid (C18:1), Linoleic Acid (C18:2. Palmitic Acid (C16:0), Stearic Acid (C18:0), Linolenic Acid (C18:3)(specifically alpha-Linolenic Acid), panthenol, tocopheryl acetate, retinol, lecithin, disodium EDTA, mastic acid, and tannins tri-, tetra-, and pentacyclic triterpene acids and alcohols including isopropyl alcohol, styrene, cinnamyl alcohol and α-pinene.

4. Composition according to claim 1, wherein: the insoluble ultraviolet absorbers are (Sr,Ca)AlSiN3 which is doped by Eu, and/or GdYEu(MoO4)3 which is doped by Eu and Sm and/or Mg14Ge5O24 which is doped by Mn, CaAlSiN3 which is doped by Eu, and/or, (LaY)3Si6N11 which is doped by Ce and/or Lu3Al5O12 which is doped by Ce or, their blends,

5. Composition according to claim 1, wherein the size of the phosphors may be in the range of 100 nm-50.0 µm.

6. Composition according to claim 1, wherein the amount of the CaAlSiN3 or (Sr Ca)AlSiN3 is in the range of %0.001-%10.000 by weight of the total composition.

7. Composition according to claim 1, wherein the oxidation states of the Eu and Sm in the GdYEu(MoO4)3 are Eu3+and Sm 3+, respectively.

8. Composition according to claim 1, wherein the amount of the GdYEu(MoO4)3 is in the range of %0.001-%1.000 by weight of the total composition.

9. Composition according to claim 1, wherein the amount of the Mg14Ge5O2 is in the range of %0.001-%1.000 by weight of the total composition.

10. Composition according to claim 1, wherein the amount of the Ce doped Lu3Al5O12, or Ce doped (LaY)3Si6N11 is in the range of %0.001-%1.000 by weight of the total composition.

11. Composition according to claim 1, wherein the amount of the mastic acid, and tri-, tetra-, and pentacyclic triterpene acids and alcohols, more specifically α-terpineol, p-cymene-8-ol, myrtenal, p-cymene, limonene, (E)-carveol, 2-undecanone, α-and β-caryophyllene, caryphyllene oxide and (E)-Me isoeugenol, is in the range of %0.001-%1.000 by weight of the total composition.

12. Composition, according to claim 1, wherein the amount of the Oleic Acid (C18:1), Linoleic Acid (C18:2. Palmitic Acid (C16:0), Stearic Acid (C18:0), Linoleic Acid (C18:3) (specifically alpha-Linolenic Acid), is in the range of %0.001-%.5.000 by weight of the total composition.

13. Composition according to claim 1, contains retinol, tocopheryl acetate and tannins tri-, tetra-, and pentacyclic triterpene acids.

14. Composition according to claim 1, further contains oils, film formers, antioxidants, antibacterial agents, thickeners including orientalis sweet gum, emulsifiers including lecithin, stabilizing and pH adjusting agents including EDTA, softeners and humectants including dexpanthenol.

15. Composition according to claim 1, is in the form of a colored cream, a milk, solid stick, a lotion, serum or spray.

## Patentansprüche

1. Sonnenschutzzusammensetzung, **dadurch gekennzeichnet, dass** sie mindestens einen Phosphor, ausgewählt aus Eu-Sm dotiertem GdYEu(MoO4)3, Mn dotiertem Mg14Ge5O24, Eu-dotiertem CaAlSiN3, Eu-dotiertem (Sr, Ca)AlSiN3, Ce dotiertem (LaY)3Si6N11, Ce dotiertem Lu3Al5O12 oder deren Mischungen, und mindestens einen Arzneiträger enthält.

2. Zusammensetzung einer lichtbeständigen Sonnenschutzemuslion gemäß Anspruch 1, wobei die aktiven Sonnenschutzagens enthaltend, aus Eu dotiertem (Sr,Ca) AlSiN3, und/oder Eu und Sm dotiertem GdYEu(MoO4)3, und Mn dotiertem Mg14Ge5O24, Eu dotiertem CaAlSiN3, und/oder Ce dotiertem (LaY)3Si6N11 oder Ce dotiertem Lu3Al5O12 oder deren Mischungen, sofort aktiviert werden, wenn sie dem Sonnenlicht ausgesetzt werden.

3. Sonnenschutzformulierung gemäß Anspruch 1, wobei die kosmetischen Arzneiträger, ausgewählt aus natürlichen Ölen, Verdickungsmitteln, Emulgatoren, Strukturierungsmitteln und Feuchthaltemitteln, entionisiertes Wasser, NatriumCarboxymethylcellulose (CMC), Glycerin, Cetylalkohol und Glycerylstearat, Ölsäure (C18:1), Linolsäure (C18:2), Palmitinsäure (C16:0), Stearinsäure (C18:0), Linolensäure (C18:3) (insbesondere alpha-Linolensäure), Panthenol, Tocopherylacetat, Retinol, Lecithin, Dinatrium-EDTA, Mastixsäure und Tannine, tri-, tetra- und pentazyklische Triterpenzusätze und Alkohole einschließlich Isopropylalkohol, Styrol, Zimtalkohol und α-Pinen enthalten.

4. Zusammensetzung gemäß Anspruch 1, wobei: die unlöslichen Ultraviolettabsorber, aus Eu dotiertem (Sr,Ca)AlSiN3, und/oder aus Eu und Sm dotiertem GdYEu(MoO4)3 und/oder aus Mn dotiertem Mg14Ge5O24, aus Eu dotiertem CaAlSiN3, und/oder aus Ce dotiertem (LaY)3Si6N11 und/oder aus Ce dotiertem Lu3Al5O12 oder deren Mischungen ist.

5. Zusammensetzung gemäß Anspruch 1, wobei die Größe des Phosphors im Bereich von 100 nm-50.0 µm liegen kann.

6. Zusammensetzung gemäß Anspruch 1, wobei die Menge von CaAlSiN3 oder (Sr, Ca)AlSiN3 im Bereich von 0.001- 10.000 Gew.-% der Gesamtzusammensetzung liegt.

7. Zusammensetzung gemäß Anspruch 1, wobei die Oxidationszustände der Eu und Sm in GdYEu(MoO4)3, Eu3+ bzw. Sm 3+ sind.

8. Zusammensetzung gemäß Anspruch 1, wobei die Menge des GdYEu(MoO4)3 im Bereich von 0.001-1.000 Gew.-% der Gesamtzusammensetzung liegt.

9. Zusammensetzung gemäß Anspruch 1, wobei die Menge des Mg14Ge5O2 im Bereich von 0.001-1.000 Gew.-% der Gesamtzusammensetzung liegt.

10. Zusammensetzung gemäß Anspruch 1, wobei die Menge des aus Ce dotiertem Lu3Al5O12, oder aus Ce dotiertem (LaY)3Si6N11 im Bereich von 0.001-1.000 Gew.-% der Gesamtzusammensetzung liegt.

11. Zusammensetzung gemäß Anspruch 1, wobei die Menge der Mastixsäure und der Tri-, Tetra- und Pentacydotriterpensäuren und -alkohole, insbesondere α-Terpineol, p-Cymen-8-ol, Myrtenal, p-Cymen, Limonen, (E)-Carveol, 2-Undecanon, α- und β-Caryophylien, Caryphylidenoxid und (E)-Me-Isoeugenol, im Bereich von 0.001-1.000 Gew.-% der Gesamtzusammensetzung liegt.

12. Zusammensetzung gemäß Anspruch 1, wobei die Menge der Ölsäure (C18:1), Linolsäure (C18:2), Palmitinsäure (C16:0), Stearinzusatz (18:0), Linolzusatz (C18:3) (insbesondere alpha-Linolensäure) im Bereich von 0.001-5.000 Gew.-% der Gesamtzusammensetzung liegt.

13. Zusammensetzung gemäß Anspruch 1, enthält Retinol, Tocopherylacetat und Tannine tri-, tetra- und pentazyklische Triterpensäuren.

14. Zusammensetzung gemäß Anspruch 1, enthält ferner Öle, Filmbildner, Antioxidantien, antibakterielle Mittel, Verdickungsmittel einschließlich Orientalis-Süßgummi, Emulgatoren einschließlich Lecithin, Stabilisierungs- und pH-Einstellmittel einschließlich EDTA, Weichmacher und Feuchthaltemittel einschließlich Dexpanthenol.

15. Zusammensetzung gemäß Anspruch 1, ist in Form einer farbigen Sahne, einer Milch, eines festen Stabs, einer Lotion, eines Serums oder Sprays.

## Revendications

1. Composition d'écran solaire **caractérisée en ce qu'**elle comprend au moins un luminophore choisi parmi GdYEu(MoO4)3 dopé par Eu-Sm, Mg14Ge5O24 dopé par Mn, CaAlSiN3 dopé par Eu, (Sr,Ca)AlSiN3 dopé par Eu, (LaY)3Si6N11 dopé par Ce, Lu3Al5O12 dopé par Ce ou leurs mélanges et au moins un excipient.

2. Composition d'émulsion d'écran solaire photostable selon la revendication 1, dans laquelle les agents d'écran solaire actifs comprenant (Sr,Ca) AlSiN3 qui est dopé par Eu, et/ou GdYEu(MoO4)3 dopé par Eu et Sm, et Mg14Ge5O24 dopé par Mn, CaAlSiN3 dopé par Eu, et/ou (LaY)3Si6N11 dopé par Ce ou Lu3Al5O12 dopé par Ce ou leurs mélanges sont immédiatement activés lors de l'exposition à la lumière du soleil.

3. Formulation d'écran solaire selon la revendication 1 dans laquelle les excipients cosmétiques sélectionnés parmi les huiles naturelles, les épaississants, les émulsifiants, les agents structurants et les humectants comprennent l'eau désionisée, la carboxyméthylcellulose sodique (CMC), la glycérine, l'alcool cétylique et le stéarate de glycéryle, l'acide oléique (C18:1), l'acide linoléique (C18:2), l'acide palmitique (C16:0), l'acide stéarique (C18:0), l'acide linolénique (C18:3) (plus précisément l'acide alpha-linolénique), le panthénol, l'acétate de tocophéryle, le rétinol, la lécithine, l'EDTA disodique, l'acide mastique et les tanins tri-, tétra- et pentacycliques triterpènes et les alcools, y compris l'alcool isopropylique, le styrène, l'alcool cinnamylique et a-pinène.

4. Composition selon la revendication 1, dans laquelle: les absorbeurs d'ultraviolets insolubles sont le (Sr,Ca)AlSiN3 qui est dopé par Eu, et/ou le GdYEu(MoO4)3 qui est dopé par Eu et le Sm et/ou le Mg14Ge5O24 qui est dopé par Mn, le CaAlSiN3 qui est dopé par Eu, et/ou le (LaY)3Si6N11 qui est dopé par Ce et/ou le Lu3Al5O12 qui est dopé par Ce ou, leurs mélanges.

5. Composition selon la revendication 1, dans laquelle la taille des phosphores peut être comprise entre 100 nm-50.0 µm.

6. Composition selon la revendication 1, dans laquelle la quantité de CaAlSiN3 ou de (Sr, Ca)AlSiN3 est comprise entre 0.001%-10.000% en poids de la composition totale.

7. Composition selon la revendication 1, dans laquelle les états d'oxydation de l'Eu et du Sm dans le GdYEu(MoO4)3 sont respectivement Eu3+ et Sm 3+.

8. Composition selon la revendication 1, dans laquelle la quantité de GdYEu(MoO4)3 est comprise entre 0.001%-1.000% en poids de la composition totale.

9. Composition selon la revendication 1, dans laquelle la quantité de Mg14Ge5O2 est comprise entre 0.001%-1.000% en poids de la composition totale.

10. Composition selon la revendication 1, dans laquelle la quantité de Lu3Al5O12 dopé au Ce, ou de (LaY)3Si6N11 dopé au Ce, est comprise entre 0.001%-1.000% en poids de la composition totale.

11. Composition selon la revendication 1, dans laquelle la quantité d'acide mastique et d'acides et d'alcools tri-, tétra- et pentacycliques triterpéniques, plus particulièrement α-terpinéol, le p-cymène-8-ol, le myrtenal, le p-cymène, le limonène, le (E)-carveol, la 2-undécanone, α-et β-caryophyliène, l'oxyde de caryophyliène et le (E)-Me isoeugenol est comprise entre %0.001-1.000% en poids de la composition totale.

12. Composition selon la revendication 1, dans laquelle la quantité d'acide oléique (C18:1), d'acide linoléique (C18:2), d'acide palmitique (C16:0), d'acide stéarique (C18:0), d'acide linoléique (C18:3) (plus précisément d'acide alpha-linolénique) est comprise entre 0.001%-5.000% en poids de la composition totale.

13. Composition selon la revendication 1, contient du rétinol, de l'acétate de tocophéryle et des tanins tri-, tétra- et pentacycliques triterpènes.

14. Composition selon la revendication 1, contient en outre des huiles, des agents filmogènes, des antioxydants, des agents antibactériens, des épaississants y compris la gomme sucrée orientalis, des émulsifiants y compris la lécithine, des agents de stabilisation et d'ajustement du pH y compris l'EDTA, des adoucissants et des humectants y compris le dexpanthénol.

15. Composition selon la revendication 1, est sous forme de crème colorée, de lait, de bâton solide, de lotion, de sérum ou de spray.
